# EUROPÄISCHE PATENTSCHRIFT

(11) **EP 0 588 022 B1**
(45) Veröffentlichungstag und Bekanntmachung des Hinweises auf die Patenterteilung: **25.09.1996**
(21) Anmeldenummer: 93111704.8
(22) Anmeldetag: 21.07.1993
(51) Int. Cl.: A61F 2/34

(54) **Gelenkpfanne für eine Hüftgelenk-Endoprothese**
Acetabular cup for a hip joint endoprosthesis
Coque acétabulaire pour une endoprothèse de l'articulation de la hanche

(30) Priorität: 15.09.1992 DE 9212419 U
(43) Veröffentlichungstag der Anmeldung: 23.03.1994
(73) Patentinhaber: Waldemar Link (GmbH & Co.), D-22315 Hamburg (DE)
(72) Erfinder: Keller, Arnold, D-23863 Kayhude (DE)
(74) Vertreter: Glawe, Delfs, Moll & Partner

(56) Entgegenhaltungen:
- EP-A- 0 239 485
- EP-A- 0 340 175
- EP-A- 0 470 912
- WO-A-84/03432
- FR-A- 2 649 005
- FR-A- 2 676 172
- US-A- 4 159 544
- US-A- 4 904 265

## Beschreibung

Die Erfindung betrifft eine Gelenkpfanne für eine Hüftgelenk-Endoprothese, bestehend aus einer in den Hüftknochen einzusetzenden Fassung und einem die Gelenkfläche bildenden Einsatz, der in der Fassung mittels eines Bajonettverschlusses verriegelbar ist.

Bei einer bekannten Gelenkpfanne dieser Art (EP-B 0 137 040) ist der Bajonettverschluß mittels einer Schraube fixierbar, die in die einander gegenüberstehenden Ränder der Fassung und des Einsatzes eingreift. Nachteilig ist bei dieser Lösung, daß die Schraube als kleines und gesondertes Teil nach dem Implantieren der Prothesenbestandteile eingesetzt werden muß, was unter Operationsbedingungen zu Schwierigkeiten führen kann, und daß diejenige Drehstellung des Einsatzes zur Fassung, in der die Schraublöcher miteinander fluchten, schwer zu finden ist. Auch ist die Schraube im montierten und implantierten Zustand nicht gesichert.

Die Erfindung vermeidet diese Nachteile durch die Merkmale des Anspruchs 1 sowie vorzugsweise des Anspruchs 2.

Die Langvertiefung in dem einen der beiden Ränder bildet zusammen mit der Schraube in dem anderen der beiden Ränder einen Anschlag, der die Verriegelungsstellung des Bajonettverschlusses angibt. Da dieser Anschlag (genauer gesagt: die Mittellinie der Schraube in der Anschlagstellung) mit der zur Schraube passenden Bohrung fluchet, die zwecks Fixierung der Verriegelungsstellung die Schraube aufnimmt, braucht die Stellung, in der die Schraube in die Sicherungsstellung überführbar ist, nicht erst gesucht zu werden, sondern ergibt sich ohne weiteres in der Anschlagstellung. Da die Schraube als Anschlagelement bereits vormontiert ist, braucht sie auch nicht erst eingesetzt zu werden, sondern nur noch aus der nicht-sichernden Stellung in die sichernde Stellung innerhalb der ihr zugeordneten Bohrung durch entsprechende Drehung überführt zu werden. Das Einsetzen der Prothese wird dadurch beträchtlich erleichtert. Auch wird die Schraube durch die erfindungsgemäße Anordnung zwangsläufig gesichert. Sie wird nämlich durch den Grund der Langvertiefung daran gehindert, gänzlich aus der vorgesehenen Position herauszutreten.

Zweckmäßigerweise ist die Langvertiefung im Rand des Einsatzes vorgesehen, während die Schraube im Rand der Fassung vormontiert ist. Jedoch ist auch der umgekehrte Fall denkbar.

Wenn die Langvertiefung im Rand des Einsatzes vorgesehen ist, besitzt die für die Aufnahme der Schraube (in diesem Fall des Schraubenkopfes) vorgesehene Bohrung eine nach außen durchtretende Fortsetzung geringeren Durchmessers, durch die ein Schraubendrehwerkzeug zum Überführen der Schraube aus der Anschlagstellung in die Sicherungsstellung überführbar ist.

Die Erfindung wird im folgenden näher unter Bezugnahme auf die Zeichnung erläutert, die ein vorteilhaftes Ausführungsbeispiel veranschaulicht. Darin zeigen:

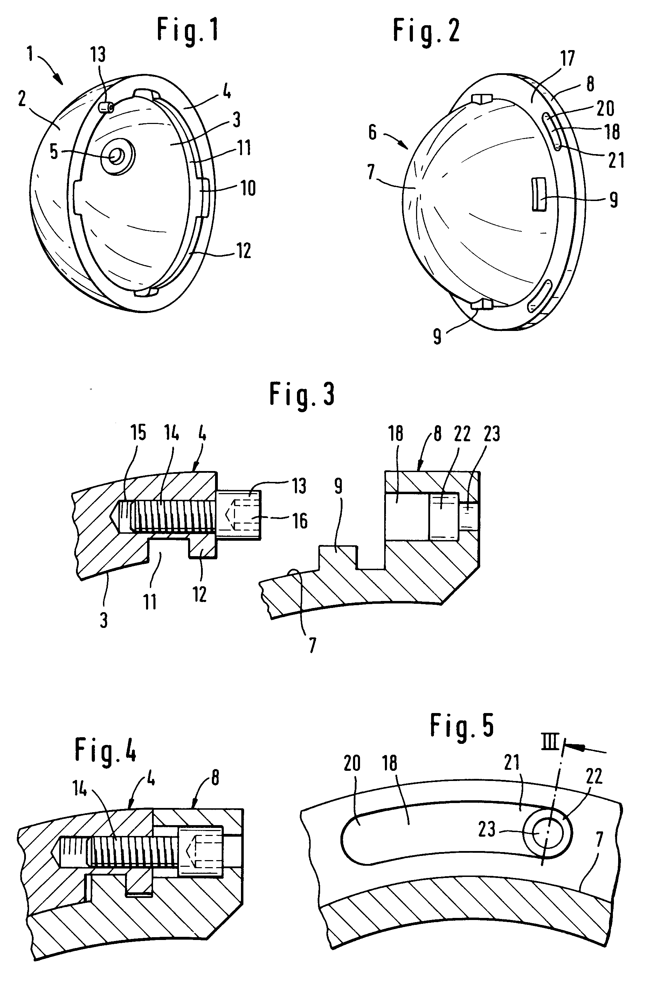

- Fig. 1 u. 2: perspektivische Ansichten der einander zugekehrten Seiten der Fassung und des Einsatzes,
- Fig. 3 u. 4: Teilschnitte in vergrößertem Maßstab der Ränder der Fassung und des Einsatzes im getrennten und im zusammengesetzten Zustand und
- Fig. 5: eine Teilansicht der Unterseite des Randes des Einsatzes.

Die Fassung 1, die aus Metall bestehen kann, hat eine zur Verankerung im Knochen in bekannter Weise gestaltete Außenfläche 2, eine sphärische Innenfläche 3 und einen Rand 4. Es können an geeigneter Stelle Bohrungen 5 zur zusätzlichen Schraubverankerung im Knochen vorgesehen sein.

Der Einsatz 6 hat eine zur Innenfläche 3 der Fassung 1 passende, sphärische Außenfläche 7 und einen flanschartig vorkragenden Rand 8 sowie eine sphärische Innenfläche zur Aufnahme einer Gelenkkugel.

Die Ränder 4 bzw. 8 der Fassung und des Einsatzes sind mit zusammenwirkenden Befestigungseinrichtungen versehen, die von über den Umfang verteilten Bajonettvorsprüngen 9 am Einsatz, radialen Vertiefungen 10 für den Durchlaß der Bajonettvorsprünge 9 am Innenumfang des Randes 4 der Fassung 1 und einer Umfangsnut 11 gebildet sind, die im Verriegelungszustand des Bajonettverschlusses die Bajonettvorsprünge 9 aufnimmt und öffnungsseitig durch einen Bund 12 begrenzt wird, hinter den die Vorsprünge 9 passend greifen und der im Bereich der Vertiefungen 10 für den Durchlaß der Vorsprünge 9 ausgespart ist.

In der Stirnfläche des Fassungsrands 4 ragt der Kopf 13 einer Schraube 14 vor, die in einer Gewindebohrung 15 im Fassungsrand 4 gehalten ist und eine Schlüsselangriffsfläche vorzugsweise in der Gestalt eines Innensechskants 16 aufweist.

Der Rand 8 des Einsatzes weist an mehreren Stellen über den Umfang verteilt in der gleichen Anzahl wie die Bajonettvorsprünge 9 und die Ausnehmungen 10 in der Stirnseite 17, die der Stirnfläche des Fassungsrands 4 gegenübersteht, in Umfangsrichtung verlaufende Langvertiefungen 18 auf, deren Weite und Tiefe den entsprechenden Abmessungen des Schraubenkopfs 13 angepaßt ist, so daß dieser darin Aufnahme finden kann. Das eine Ende 20 jeder Langvertiefung übergreift jeweils diejenige Stelle, in der sich im Fassungsrand 4 der Schraubenkopf 13 beim Einsetzen des Einsatzes in die Fassung befinden kann, wenn die Bajonettvorsprünge 9 sich an derselben Stelle wie die Bajonettausnehmungen 10 befinden. Das andere Ende 21 der Langvertiefungen 18 befindet sich an derjenigen Stelle, wo sich im gewünschten Verriegelungszustand, wenn die Vorpsrünge 9 hinter dem Bund 12 greifen, der Schraubenkopf 13 befindet. Das Ende 21 der Langvertiefungen bildet daher zusammen mit dem Schraubenkopf 13 einen Anschlag, der die Verriegelungsstellung des Einsatzes in der Fassung bestimmt.

Am Ende 21 der Langvertiefung 18 ist eine Bohrung 22 vorgesehen, deren Wandung mit dem Ende der Langvertiefung fluchtet und die zur passenden Aufnahme des Schraubenkopfs 13 bemessen ist. Wenn der Einsatz in der Fassung in die Verriegelungsstellung gedreht ist, in der der Schraubenkopf 13 am Ende der Langvertiefung 18 anschlägt, kann der Schraubenkopf demnach ein wenig herausgedreht werden, um in die Bohrung 22 einzudringen und dort die Drehfixierung des Einsatzes gegenüber der Fassung zu bewirken. Dafür ist eine Bohrung 23 vorgesehen, durch die ein Schraubendrehwerkzeug, beispielsweise ein Innensechskantschlüssel gesteckt werden kann. Die Bohrung 23 hat einen geringeren Durchmesser als der Schraubenkopf 13. Ihre Verengung gegenüber der Bohrung 22 bewirkt somit eine Sicherung der Schraube 14 an Ort und Stelle.

Zum Einsetzen und Sichern des Einsatzes in der Fassung ist es daher lediglich erforderlich, den Bajonettverschluß in die Verriegelungsstellung zu bringen, bis der Schraubenkopf 13 am Ende der Langvertiefung anschlägt, um dann die Schraube ein wenig herauszudrehen, so daß sie hinreichend in die Bohrung 22 eintritt.

## Patentansprüche

1. Gelenkpfanne für eine Hüftgelenk-Endoprothese, bestehend aus einer in den Hüftknochen einzusetzenden Fassung (1) und einem die Gelenkfläche bildenden Einsatz (6), der in der Fassung mittels eines Bajonettverschlusses verriegelbar ist, der durch eine Schraubsicherung an den Rändern (4,8) der Fassung und des Einsatzes fixierbar ist, dadurch gekennzeichnet, daß die Schraubsicherung in dem einen Rand (8) eine dem anderen Rand (4) zugewendete, in Umfangsrichtung verlaufende Langvertiefung (18) umfaßt und an dem anderen Rand (4) eine Schraube (14) vorgesehen ist, die in die Langvertiefung (18) eingreift und mit einem Ende (21) der Langvertiefung einen Anschlag in der Verriegelungsstellung bildet, wobei fluchtend mit diesem Ende (21) der Langvertiefung (18) im Grund der Langvertiefung eine zu der Schraube passende Bohrung (22) vorgesehen ist, in die die Schraube (14), die Verriegelungsdrehstellung sichernd, einschraubbar ist.

2. Gelenkpfanne nach Anspruch 1, dadurch gekennzeichnet, daß an die zur Aufnahme des Schraubenkopfes (13) bemessene Bohrung (22) im Rand (8) des Ansatzes (6) ein Bohrungsabschnitt (23) geringeren Durchmessers, hinreichend für den Durchtritt eines Schraubendrehwerkzeugs, anschließt.

## Claims

1. An acetabular cup for a hip joint endoprosthesis, comprising a socket (1) to be inserted in the hip bone and an insert (6) which forms the joint surface and which can be locked in the socket by means of a bayonet fastening which can be fixed by a screw locking means at the rims (4,8) of the socket and of the insert, characterised in that the screw locking means in one rim (8) comprises a slot (18) facing the other rim (4) and extending in circumferential direction, and on the other rim (4) a screw (14) is provided which engages in the slot (18) and which at one end (21) of the slot forms a stop member in the locking position, wherein in alignment with this end (21) of the slot (18) a bore (22) is provided in the bottom of the slot (18), which bore fits the screw and into which the screw (14) can be screwed, thereby securing the rotational locking position.

2. An acetabular cup according to Claim 1, characterised in that a bore portion (23) of smaller diameter, which is adequate for the passage of a screwdriver, adjoins the bore (22) in the rim (8) of the insert (6), which bore is designed to accommodate the screw head (13).

## Revendications

1. Coque acétabulaire pour une endoprothèse de l'articulation de la hanche, se composant d'une monture (1) destinée à être mise en place dans l'os iliaque et d'une pièce rapportée (6) qui constitue la surface articulaire et qui est verrouillable dans la monture au moyen d'un joint à baïonnette qui peut être fixé par un système de sécurité à vis sur les bords (4,8) de la monture et de la pièce rapportée, caractérisée en ce que le système de sécurité à vis comprend, dans l'un des bords (8), une cavité oblongue (18) dirigée vers l'autre bord (4) et s'étendant dans la direction circonférentielle, et en ce qu'il est prévu, sur l'autre bord (4), une vis (14) qui pénètre dans la cavité oblongue (18) et forme, avec une extrémité (21) de la cavité oblongue, une butée dans la position de verrouillage, et il est prévu, dans l'alignement de cette extrémité (21) de la cavité oblongue (18), dans le fond de celle-ci, une forure (22) adaptée à la vis, dans laquelle la vis (14) peut être engagée par vissage pour fixer la position angulaire de verrouillage.

2. Coque acétabulaire selon la revendication 1, caractérisée en ce qu'une partie de forure (23) de diamètre plus petit, suffisant pour le passage d'un tourne-vis, fait suite à la forure (22), dimensionnée de manière à recevoir la tête (13) de la vis, dans le bord (8) de la pièce rapportée (6).
